# EUROPÄISCHE PATENTSCHRIFT

(11) **EP 3 219 265 B1**
(45) Veröffentlichungstag und Bekanntmachung des Hinweises auf die Patenterteilung: **26.01.2022**
(21) Anmeldenummer: 15855902.1
(22) Anmeldetag: 30.10.2015
(51) Int. Cl.: A61B 17/221, A61B 17/00

(54) **VORRICHTUNG ZUR MANIPULATION VON FREMDKÖRPERN IN HOHLORGANEN (VARIANTEN)**
DEVICE FOR MANIPULATING FOREIGN BODIES IN HOLLOW ORGANS (VARIANTS)
DISPOSITIF DE MANIPULATION DE CORPS ÉTRANGERS DANS DES ORGANES CREUX (VARIANTES)

(30) Priorität: 31.10.2014 RU 2014144355
(43) Veröffentlichungstag der Anmeldung: 20.09.2017
(73) Patentinhaber: Obschestvo S Ogranichennoy Otvetstvennostyu "Smet", Tomsk 634021 (RU)
(72) Erfinder: HACHIN, Vladimir Nikolaevich, Tomsk 634000 (RU); HACHIN, Stepan Vladimirovich, Tomsk 634000 (RU)
(74) Vertreter: Jeck, Anton
(86) Internationale Anmeldenummer: PCT/RU2015/000726
(87) Internationale Veröffentlichungsnummer: WO 2016/068758

(56) Entgegenhaltungen:
- EP-A2- 0 914 807
- WO-A1-2009/074981
- WO-A1-2010/010545
- WO-A1-2016/040172
- RU-C1- 2 466 690
- US-A- 2 556 783
- US-A- 5 192 286
- US-A1- 2008 033 467
- US-A1- 2014 263 032

## Beschreibung

Die Erfindung gehört zum medizinischen Bereich, und zwar zu den medizinischen Instrumenten für endoskopische Chirurgie, und kann für das Herausziehen von Fremdkörpern, z. B. von Harn- und Gallensteinen, Thromben, Polypen, aus Hohl- und Rohrorganen sowie bei anderen Manipulationen mit Fremdobjekten angewendet werden.

Aus der US2943626 ist eine Einrichtung für das Herausziehen von Fremdkörpern aus dem Organismus eines Menschen oder Tieres, z. B. Steinen aus Harnleitern, bekannt. Die Einrichtung ist als Extrakteur oder Dormia-Körbchen bekannt und besteht aus einem Katheter (Schlauch) und einem Führungsdraht mit einem Körbchen-Abscheider aus Federelementen, das am distalen Ende eines Führungsdrahts untrennbar befestigt ist. Der Führungsdraht mit dem Körbchen-Abscheider bewegt sich frei im Katheter. Bei dem Einfahren und Herausfahren des Körbchens aus dem Katheter verringert oder vergrößert sich seine Größe entsprechend, wobei das Körbchen in eine zusammengerollte Transportposition für das Zubringen zum Fremdkörper oder in eine entfaltete Arbeitsposition für das Greifen und Entfernen des Fremdkörpers gebracht wird. Seit sechzig Jahren kennen die Fachleute diese Einrichtung; sie wird ohne wesentliche Änderungen in der klinischen Weltpraxis noch heute aktiv angewendet.

Zu den Nachteilen dieser Einrichtung gehören niedrige Extraktionseigenschaften des Körbchen-Abscheiders, die sich auf ein Operationsprozedere "Lithoextraktion" beschränken. Die symmetrische Konstruktion der proximalen und distalen Teile des Körbchen-Abscheiders können ein sicheres Greifen und Festhalten des Fremdkörpers bei der Extraktion nicht gewährleisten. Die Einrichtung sieht kein zusätzliches Prozedere im Operationsfeld des Fremdkörpers vor, das sehr oft erforderlich ist, z. B. eine Drainage des Harnleiters und der Niere, die Einführung von Schmerzmitteln und Schmiermitteln, die Durchführung einer Lithotrepsie innerhalb des Körbchens usw.

Es sind mehrere Versuche zur Verbesserung der Extraktionseigenschaften des Dormia-Körbchens und der Erweiterung der Operationsmöglichkeiten der Einrichtung bekannt.

Die Originaleinrichtung für das Herausziehen der Fremdkörper aus den physiologischen Kanälen oder Kanälen im menschlichen Körper, die vom gleichen Autor angeboten ist, hat zwei Dormia-Körbchen, die ineinander eingesteckt sind (US4612931). Nach der Meinung des Erfinders ist diese Einrichtung weniger traumatisch, aber ihre Extraktionseigenschaften - Möglichkeit des Greifens, insbesondre eines Großobjekts in das interne Körbchen - sind noch schlechter als bei der Einrichtung mit einem Körbchen. Außer der geringeren Traumatisierung hat die Einrichtung keine weiteren Vorteile.

Durch die US5057114 ist ferner eine Einrichtung für das Herausziehen von Fremdkörpern bekannt, die die gleichen Elemente hat: einen Katheter und einen Führungsdraht mit einem Körbchen-Abscheider am distalen Ende. Die Einrichtung ist im Vergleich zu der vorherigen dadurch gekennzeichnet, dass sie am distalen Ende keinen Körbchen-Abscheider aufweist. Die Abwesenheit einer Spitze ermöglicht es, die klein dimensionierten Steine an den unteren Wänden der Harn- oder Gallenblase zu greifen, wobei innere Wände des Hohlorgans nicht verletzt werden. Aber die Einrichtung gewährleistet kein sicheres Greifen von größeren Steinen und bei Bedarf keine sichere Befreiung vom Fremdkörper. Außerdem sieht die Konstruktion der Einrichtung keine Durchführung einer zusätzlichen Manipulation mit dem Objekt innerhalb des Körbchens vor, z. B. keine Lithotripsie der großen Steine.

Aus der US5989266 ist eine Einrichtung für das Herausziehen von Fremdkörpern bekannt, die unter gleichen Grundsätzen, wie oben beschrieben, gebaut ist und die einen ähnlichen Körbchen-Abscheider in Form von mehreren Schlingen verschiedener Größen ohne Spitze am distalen Ende der Drahtführung aufweist. Die Einrichtung stellt sich als minimal invasives Gerät für das Greifen und Herausziehen der Harnsteine meistens aus dem Nierenkelch dar. Der Körbchen-Abscheider der Einrichtung ist einfach in der Herstellung, hat eine erhöhte Radialsteifigkeit (Dilatation), hat dabei aber Nachteile, die für Drahtkonstruktionen solcher Art üblich sind: eine Unsicherheit des Greifens und Festhaltens des Steins bei der Extraktion und die Abwesenheit der Möglichkeit der Anwendung von anderen Instrumenten für das OP-Prozedere mit den Fremdkörpern.

Aus der US6780193 ist ein chirurgischer Extrakteur bekannt, bei dem der Körbchen-Abscheider wie bei allen vorangehenden analogen Geräten mit dem distalen Ende der Drahtführung untrennbar verbunden ist, aber eine Menge von Drahtelementen im distalen Teil und eine geringe Anzahl von Drahtelementen im proximalen Teil aufweist. Eine derartige Konstruktion des Körbchen-Abscheiders erleichtert das Greifen des Objekts durch große Fenster im proximalen Teil und sein sicheres Festhalten mit der Mehrheit der Drahtelemente im distalen Teil des Körbchen-Abscheiders. Aber die erhöhte Wirksamkeit des Greifens eines derartigen Körbchens ist auch sein Nachteil: bei Bedarf kann das Körbchen nicht leicht vom Stein befreit werden. Außerdem sieht die Konstruktion der Einrichtung keine Möglichkeit vor, andere Prozedere im Operationsfeld des Objekts anzuwenden.

Durch die US7101380 ist auch eine chirurgische Einrichtung für das Herausziehen von Fremdkörpern bekannt, die eine umfassende Anwendung in der klinischen Weltpraxis der letzten Zeit gefunden hat, wesentliche Wettbewerbsvorteile im Vergleich zu den oben genannten Einrichtungen hat und die nächstliegende Lösung ist, die als Prototyp der vorliegenden Erfindung gewählt wurde. Die Einrichtung hat eine traditionelle Konstruktion: Katheter und Drahtführung mit dem Körbchen-Abscheider am distalen Ende. Aber der Körbchen-Abscheider dieser Einrichtung ist grundlegend anders, er besteht mehrheitlich aus Fäden, die im distalen Teil Schlingen aufweisen, die sich miteinander so gekreuzt und verschlungen haben, dass sie eine Netzstruktur bilden, die die kleinsten Objekte festhalten kann. Im proximalen Teil sind die Fäden so verschlungen, dass sie eine Konstruktion mit größeren Öffnungen bilden, durch die in das Körbchen sogar relativ große Objekte eindringen können. Demzufolge sind die Extraktionseigenschaften genügend hoch.

Die Nachteile der Einrichtung sind:
- die Unmöglichkeit des Greifens von größeren Objekten, die die Größe der Seitenöffnungen überschreiten, das mit der festen Größe der Seitenöffnungen im proximalen Teil des Körbchens verbunden ist;
- die Kompliziertheit der Befreiung des mit dem Abscheider gefangenen Objekts bei Bedarf, z. B. bei einer schwachen Durchgängigkeit des Harnleiters oder Gallengangs oder wegen der Struktur im Wege der Evakuierung des Steins;
- keine Möglichkeit der gleichzeitigen Durchführung anderer Manipulationen mit dem Objekt, wenn es notwendig ist, z. B. die Lithotripsie im Körbchen, die mit der Sammlung und Entfernung der Fragmente des Steins vereinigt ist, die Drainage der Niere und des Harnleiters, die Einführung von Schmerz-, Schmier- und anderen Kontrastmitteln, die Anwendung von angiographischen Instrumenten und das Prozedere im OP-Bereich des Fremdkörpers.

Aus der US 2014/0263032 A1 geht eine Vorrichtung zum Sammeln von Zellen hervor, welche mit einem länglichen Körper und einem Netzelement gebildet ist.

Die WO 2010/010545 A1 lehrt eine Vorrichtung zum Entfernen von Fremdkörpern aus der Blutbahn mit einem netzförmigen Körper, welche an einer Haltevorrichtung angeordnet ist.

Die WO 2016/040172 A1 offenbart eine Vorrichtung zum Entfernen von Fremdkörpern aus der Blutbahn an dessen Ende eine netzförmige Vorrichtung zum Auffangen der Fremdkörper angeordnet ist, wobei diese Vorrichtung über Verbindungselemente durch einen Katheter mit einer Manipulationseinrichtung verbunden ist.

Die EP 0 914 807 A2 betrifft eine Vorrichtung zum Entfernen von Thromben aus Hohlorganen. Diese ist mit einem vorderen Bereich zum Durchstoßen des Thrombus bereitgestellt und einem Sammelelement, welches ein Ableiten des durchstoßenen Thrombus bewirken soll.

In der US 5 192 286 A ist ein Katheter mit einem netzförmigen Abscheider beschrieben, der in einer Transportposition zusammengeklappt an einem, im Katheter angeordneten, Führungsdraht anliegt und während der Anwendung des Katheters mit einem zusätzlichen Hilfsmittel in eine ausgeklappte Position gebracht werden kann, wobei der Abscheider verschieblich an dem Führungsdraht angeordnet ist.

Die Aufgabe der Erfindung ist die Entwicklung einer Einrichtung für die Manipulationen mit Fremdkörpern in Hohlorganen, die ein Greifen, eine Fragmentation und ein Festhalten des Fremdkörpers und seiner Fragmente bei der Extraktion unabhängig von seiner Größe und Form ermöglicht. Bei Bedarf soll es auch möglich sein, das Objekt in jeder Etappe der Manipulation zu befreien.

Das technische Ergebnis der Erfindung ist eine Steigerung der Wirksamkeit der Manipulationen mit den Fremdkörpern durch die Verbesserung der Extraktionseigenschaften des Abscheiders des Fremdkörpers und die Erweiterung der funktionalen Möglichkeiten der Einrichtung.

Eine nichtbeanspruchte Variante der Einrichtung für die Manipulationen mit Fremdkörpern in den Hohlorganen weist einen Katheter, einen Drahtführer und einen Abscheider des Objekts auf, der mit dem Katheter und/oder Drahtführer verbunden ist, und dass der Drahtführer ausgehöhlt und nahtlos ist, wobei der Abscheider des Objekts eine Netzoberfläche mindestens in einem Teil des Abscheiders aufweist. Der Abscheider des Objekts kann mit dem Katheter und/oder mit dem Drahtführer durch eine lösbare und/oder unlösbare Verbindung verbunden sein, wobei die lösbare Verbindung mit Hilfe z. B. von Gewinden, Verschluss, Greifvorrichtung, Nocken, und die unlösbare Verbindung mit Hilfe von Schweißen, Löten, Kleben der gemeinsamen Elemente der Konstruktion der angrenzenden Teile der Einrichtung ausgeführt ist.

Außerdem , in dieser nicht beanspruchten Variante, ist der Abscheider des Objekts, Katheter und Drahtführer, ausgeführt aus
- Metall, z. B. Nitinol, Nirostahl, röntgenkontrastierende Legierung;
- Kunststoff, z. B. Polyamid, Kapron, Nylon;
- Verbundmaterial.

Der Katheter, in dieser nicht beanspruchten Variante, ist außerdem ausgeführt:
- mit Doppelwänden;
- Wände sind verschiebbar zueinander ausgeführt;
- perforiert.

Der Drahtführer, in dieser nicht beanspruchten Variante, ist außerdem ausgeführt:
- mindestens aus zwei separaten Drähten;
- zwischen den distalen Enden zweier Drähte liegt ein Abscheider;
- Drähte sind verschiebbar zueinander ausgeführt.

Der Drahtführer in dieser nicht beanspruchten Variante, liegt dabei:
- intern im Katheter;
- außerhalb des Katheters;
- zwischen den Wänden des Katheters.

Das angegebene technische Ergebnis wird dadurch erreicht, dass die Einrichtung für die Manipulationen mit Fremdkörpern in den Hohlorganen laut der Erfindung einen Kather Drahtführer und Abscheider des Objekts aufweist, wobei der Abscheider des Objekts, Katheter und Drahtführer aus einem einzigen Ausgangsdetail ausgeführt sind, der Drahtführer ausgehöhlt oder nahtlos ist und der Abscheider des Objekts mit der Netzoberfläche mindestens in einem Teil des Abscheiders angeordnet ist.

Als Ausgangsdetail kann z. B. ein Rohr, Draht oder Band angewendet werden. Außerdem ist das Ausgangsdetail ausgeführt aus:
- Metall, z. B. Nitinol, Nirostahl, röntgenkontrastierende Legierung;
- Kunststoff, z. B. Polyamid, Kapron, Nylon;
- Verbundmaterial.

In der vorliegenden Beschreibung versteht man unter der lösbaren Verbindung eine Verbindung, die jederzeit gelöst werden kann, abweichend von der zeitlich konstanten unlösbaren Verbindung. Eine unlösbare Verbindung kann durch Schweißen, Löten oder Kleben von gemeinsamen Elementen der Konstruktion der angrenzenden Teile der Einrichtung oder in jeder anderen den Fachleuten bekannten Weise ausgeführt werden. Eine lösbare Verbindung kann mit Verschluss, Gewinde, Greifvorrichtung, Nocken oder in jeder anderen den Fachleuten bekannten Weise ausgeführt werden.

Der Katheter wird durch ein Rohr gebildet, das aus Metall, Kunststoff oder Verbundmaterial, z. B. Nitinol, Nirostahl, röntgenkontrastierende Legierung, Polyamid oder ihre Mischungen, ausgeführt ist.

Der ausgehöhlte Drahtführer ist ein Drahtführer mit Lumen im Innern, d. h. mit einem Kanal jeder Form, und kann aus Metall, Kunststoff oder Verbundmaterial, z. B. Nitinol, Nirostahl, Polyamid oder Polyamid, das mit Nitinol armiert ist, bestehen. Der geschlossene Drahtführer hat kein Lumen, dabei kann der geschlossene Drahtführer metallisch, nichtmetallisch, aus Verbundmaterial, ein- oder mehradrig sein.

Die Gesamtheit der kennzeichnenden Merkmale setzt verschiedene Varianten der Ausführung und Wettbewerbsvorteile der angebotenen Einrichtung voraus, wobei umfangreiche zusätzliche Möglichkeiten angeboten werden, damit die Wirksamkeit der Manipulationen im OP-Bereich des Fremdkörpers erhöht wird.

Zum Beispiel wird mit der gleichzeitigen Verbindung des Abscheiders des Objekts mit Katheter und Drahtführer oder mit zwei Drähten des Drahtführers folgendes gewährleistet:
- das Greifen des Objekts fast jeder Größe, da die Greiffläche des Abscheiders nur durch Verschiebung des Drahtführers in Hinsicht auf die Katheter oder zwei Drähte zueinander geregelt wird, wobei die Größe der Verschiebung nicht begrenzt wird;
- sicheres Festhalten des Fremdkörpers bei der Extraktion unabhängig von der Größe und Form des Körpers mit der Netzfläche des Abscheiders und mit der Möglichkeit seiner Platzierung distal (hinten) dem Objekt;
- sichere Befreiung des Fremdkörpers aus dem Abscheider durch einfache Verschiebung der Netzfläche des Abscheiders aus der Position "hinten" in die Position "seitlich" des Objekts.

In der Variante der Ausführung mit dem ausgehöhlten Drahtführer ermöglicht die Einrichtung nach dem Greifen des Fremdkörpers in den Abscheider zusätzlich gezielt auf das Objekt, z. B. Harnstein durch den Kanal des Drahtführers die Sonde des Lithotripters zuzuführen und eine Kontaktfragmentation des im Abscheider fixierten Objekts durchzuführen, die Fragmente des Objektes zu sammeln und zu entfernen, ohne die inneren Organe zu verletzen, kontinuierlich Niere und Harnleiter zu drainieren, um die Entwicklung der mit der Harntransportstörung verbundenen Komplikationen zu vermeiden, Schmerzmittel, Kontrastmittel für die Positionierung des Steins, Schmiermittel zur Erleichterung der Traktion des Steins und seiner Fragmente einzuführen, angiographische Drahtführer für das Anbringen der Einrichtung zu dem Fremdkörper und die Darstellung der Lage des Abscheiders anzuwenden sowie andere Instrumente und Stoffe im OP-Bereich des Fremdkörpers anzuwenden. Eine Perforation des Katheters verbessert die Drainage des Hohlorgans.

Das Erfindungswesen und einige Varianten der Ausführung der Erfindung werden nun anhand von Beispielen näher erläutert. Diese Beispiele sind im Großen und Ganzen so getroffen, um die kennzeichnenden Merkmale verschiedener Varianten der Ausführung der Einrichtung zu betonen. Es zeigen:

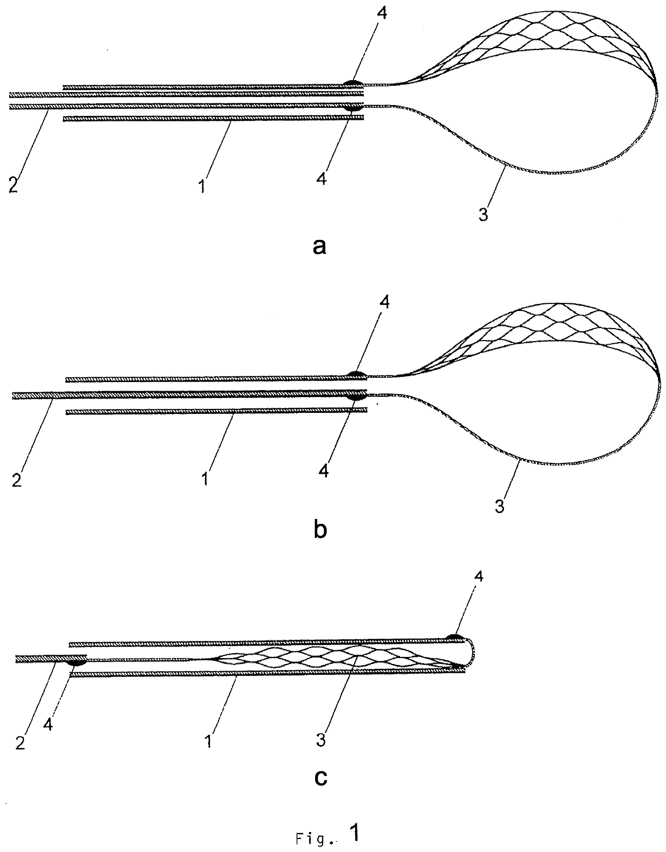

Fig. 1 eine nicht beanspruchte Ausführungsvariante der Einrichtung mit der unlösbaren Verbindung der gekoppelten Teile durch Schweißen, Löten, Kleben in den Verbindungsstellen:
   a Abscheider des Objekts mit Katheter und Drahtführer, der als ausgehöhlt ausgeführt ist;
   b Abscheider des Objekts mit Katheter und Drahtführer, der als geschlossen ausgeführt ist;
   c Abscheider des Objekts ist mit dem Drahtführer in den Katheter eingezogen, und die Einrichtung ist in den Transport-Zustand für den Transport zu dem Fremdkörper gebracht;

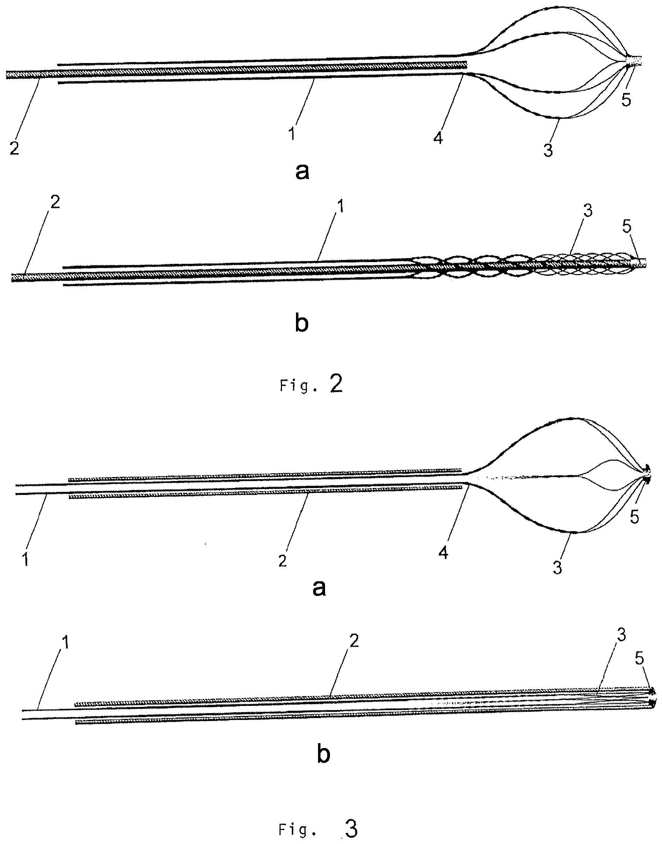

Fig. 2 eine nicht beanspruchte Ausführungsvariante der Einrichtung:
   a unlösbare Verbindung des Abscheiders des Objekts mit Katheter, die durch die für die beiden Elemente gemeinsame Konstruktion gebildet wird, z. B. Gesamtdrähte, die den Katheter armieren und den Abscheider des Objekts und geschlossenen Drahtführers bilden;
   b lösbare Verbindung des geschlossenen Drahtführers mit dem Abscheider des Objekts, die z. B. durch Nocken oder eine Greifvorrichtung von innen mit einem Element der Konstruktion am distalen Ende des Abscheiders gebildet wird; der Abscheider des Objekts ist mit dem Drahtführer in die Transportposition für das Zubringen zum Objekt zusammengebracht; der Drahtführer liegt im Innern des Katheters;
Fig. 3 eine nicht beanspruchte Ausführungsvariante der Einrichtung, bei der
   a eine unlösbare Verbindung des Abscheiders des Objekts mit dem Katheter, die durch die für die beiden Elemente gemeinsame Konstruktion gebildet wird, z. B. Gesamtdrähte, die den Katheter armieren und den Abscheider des Objekts mit einem Element der Konstruktion für die äußere Stütze des Drahtführers bilden;
   b eine lösbare Verbindung des ausgehöhlten Drahtführers mit dem Abscheider des Objekts, die z. B. durch die Stütze im Element der Konstruktion am distalen Ende des Abscheiders gebildet wird; der Abscheider des Objekts ist im Drahtführer für die Transportposition für das Zubringen zum Objekt gefaltet; der Drahtführer liegt außerhalb des Katheters;

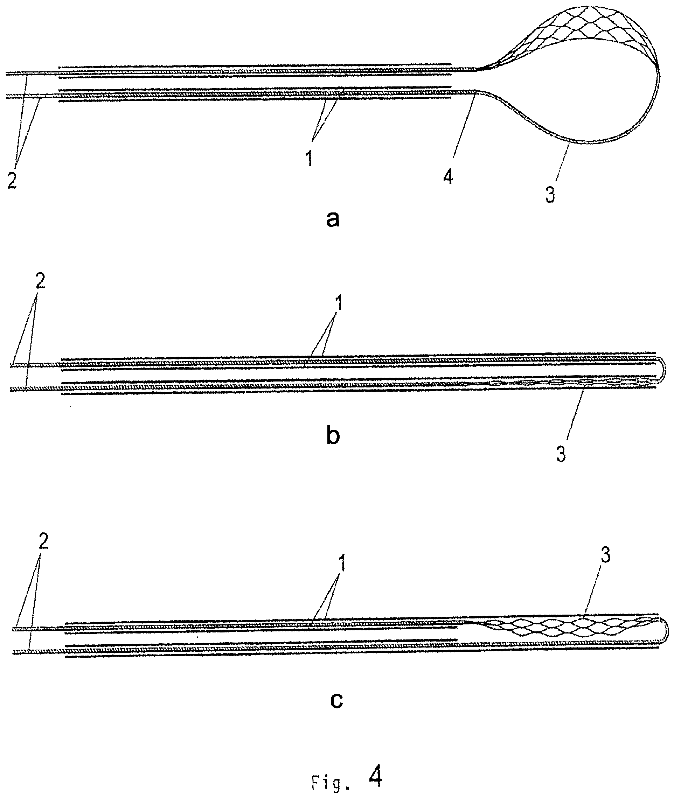

Fig. 4 eine nicht beanspruchte Ausführungsvariante der Einrichtung, bei der
   a eine unlösbare Verbindung des Abscheiders des Objekts mit zwei Drähten des Drahtführers, die zwischen den Wänden des Katheters liegen, gebildet wird;
   b der Abscheider des Objekts mit dem Drahtführer zwischen den Wänden des Katheters hineingezogen und die Einrichtung in die Transportposition für das Zubringen zum Objekt gebracht ist;
   c der Abscheider des Objekts in den äußeren Wänden des Katheters in die Transportposition für das Zubringen zum Katheter gefaltet und der Drahtführer zwischen den Wänden des Katheters angeordnet ist;
Fig. 5
   a einen Abscheider des Objekts, der aus dem distalen Ende des Katheters gebildet ist, und den Drahtführer;
   b einen Abscheider des Objekts und den Drahtführer, die aus den Gesamtdrähten bestehen, und den Katheter;

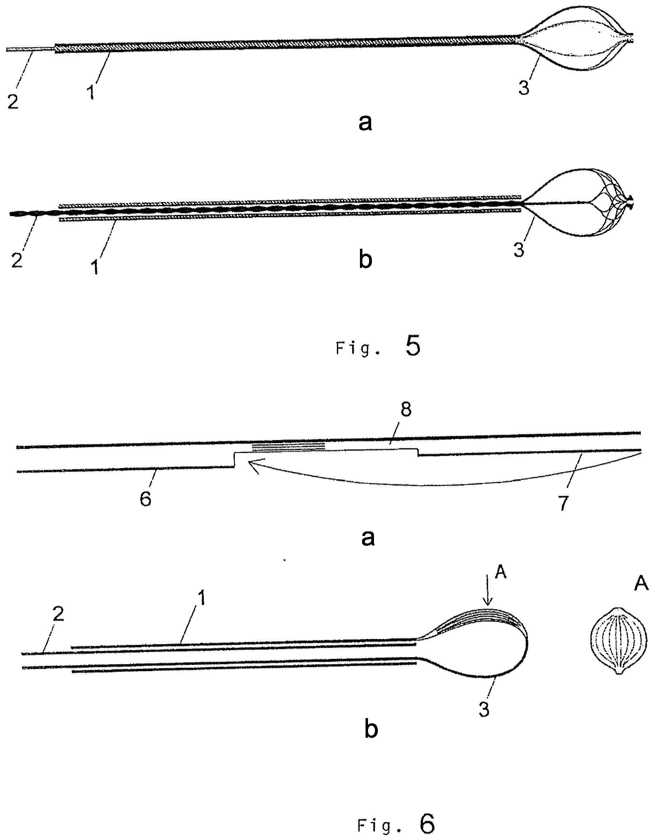

Fig. 6 ein Schema der Fertigung der Einrichtung aus einem Detail-Katheter:
   a eine Zwischenetappe der Fertigung der Einrichtung, bei der aus dem Ausgangsdetail, Katheter der gleichen Größe, Durchmesser des linken Teils von dem größer als Durchmesser des rechten Teils ist, ein Segment in seinem mittleren Teil ausgeschnitten wird, aus dem der Abscheider des Objekts gebildet wird;
   b eine zusammengebaute Einrichtung, wobei aus dem mittleren Teil des Ausgangsdetails der Abscheider des Objekts gebildet ist; der rechte Teil des Ausgangsdetails wird zum ausgehöhlten Drahtführer und wird in den linken Teil des Ausgangsdetails eingesteckt, das zum Katheter der Einrichtung wird; die Netzoberfläche des Abscheiders des Objekts ist in Fig. 6b dargestellt;

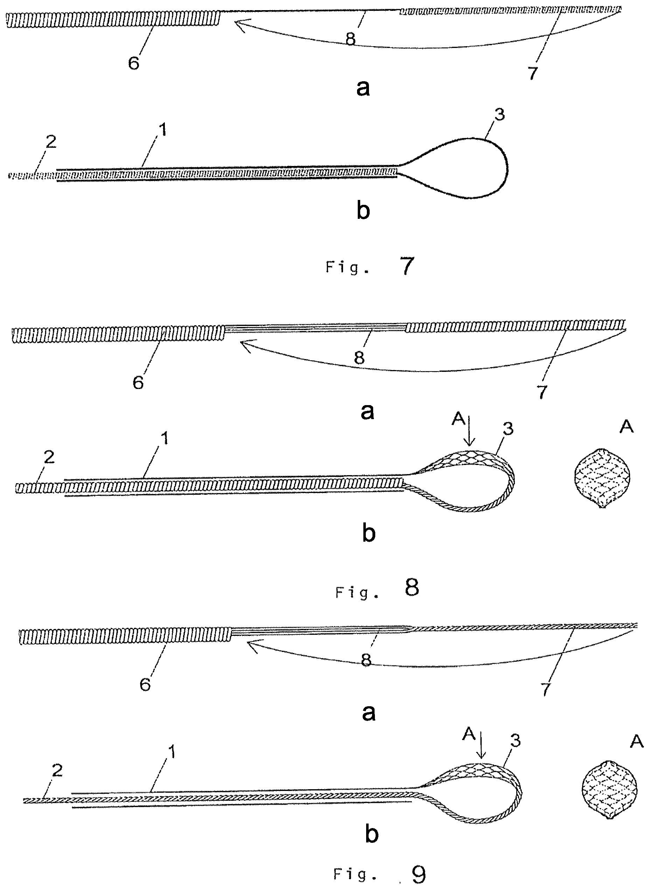

Fig. 7 ein Schema der Fertigung der Einrichtung aus einadrigem Draht:
   a eine Zwischenetappe der Fertigung der Einrichtung; aus dem einadrigen Draht wird durch seine Aufwicklung auf die Schablone der linke Katheter gebildet, danach wird durch einen Abstand, aus dem der Abscheider des Objekts gebildet wird, ein zweiter Katheter gebildet, dessen Durchmesser kleiner als der Durchmesser des Katheters ist;
   b die zusammengebaute Einrichtung, wobei die Verfahrensweise bei der Zusammensatzung der Einrichtung und der Teile des Ausgangsdetails und die Nummerierung der Elemente und Teile des Details mit der Fig. 6 übereinstimmen;
Fig. 8 ein Schema der Fertigung der Einrichtung aus einem mehradrigen Draht, wobei die Verfahrensweise der Fertigung der Einrichtung und der Nummerierung ihrer Elemente und Teile der Fig. 7 ähnlich sind; die Netzoberfläche des Abscheiders des Objekts ist anschaulich in Fig. 8b dargestellt;
Fig. 9 ein Schema der Fertigung der Einrichtung aus einem mehradrigen Draht, wobei die Verfahrensweise der Fertigung der Einrichtung und der Nummerierung ihrer Elemente und Teile der Fig. 7, 8 ähnlich sind; die Netzoberfläche des Abscheiders des Objekts ist anschaulich in Fig. 9b dargestellt;
Fig. 10 ein Schema der Variante der Funktionsweise der Einrichtung, bei der Folgendes dargestellt ist: ein Hohlorgan (Harnleiter) und Fremdkörper: Harnstein, Sonde und Fragmente des Steins;
Fig. 11 ein Schema der Variante der Funktionsweise der Einrichtung, bei der Folgendes dargestellt ist: ein Katheter, ein Abscheider des Objekts und eine Sonde.

Die Einrichtung zur Manipulation mit den Fremdkörpern in den Hohlorganen in ihrer mit einer Netzoberfläche. Der Abscheider des Objekts kann , in einer nicht beanspruchten Variante, mit dem Katheter und/oder Drahtführer unlösbar in den Verbindungsstellen 4 (Fig. 1a-c) und/oder lösbar in den Verbindungsstellen 5 (Fig. 2a, b; 3a, b) verbunden sein, und der Drahtführer kann als ausgehöhlt (Fig. 1a) oder geschlossen (Fig. 1b) ausgeführt werden, wobei die Netzoberfläche mindestens an einem Teil des Abscheiders 3 ausgeführt ist (Fig. 1a, b; 2a; 3a; 5a, b, 6b; 8b; 9b).

Die Einrichtung zur Manipulation mit den Fremdobjekten in den Hohlorganen in der Erfindung enthält einen Katheter 1, Drahtführer 2 und Abscheider des Objekts 3 mit einer Netzoberfläche. Der Abscheider des Objekts 3, der Katheter 1 und der Drahtführer 2 sind dabei aus einem Ausgangsdetail, z. B. aus einem Rohr mit verschiedenen Durchmessern in den Teilen 6 und 7 (Fig. 6a) sowie einadrigem (Fig. 7) und mehradrigem (Fig. 8, 9) Draht ausgeführt, wobei die Netzoberfläche mindestens in einem Teil des Abscheiders ausgeführt (Fig. 8b, 9b) ist. Eine Besonderheit der nicht beanspruchten Ausführungsvariante der Einrichtung, die in Fig. 1 dargestellt ist, ist die Möglichkeit der Platzierung der Netzoberfläche in verschiedenen Teilen des Schlingen-Abscheiders 3 durch Einziehen des Drahtführers 2 in den Katheter 1 oder Auswurf des Drahtführers 2 aus dem Katheter 1. Eine Besonderheit der nicht beanspruchten Ausführungsvariante der Einrichtung die in Fig. 2 dargestellt ist, ist die Möglichkeit der Entfernung des Drahtführers 2 aus dem Katheter 1 nach dem Greifen des Objekts mit dem offenen Abscheider 3 (Fig. 2a). Damit wird der Lumen des Katheters 1 für die Arbeit eines anderen Instruments befreit.

Eine Besonderheit der nicht beanspruchten Ausführungsvariante der Einrichtung, die in Fig. 3a dargestellt ist, ist die Möglichkeit der festen räumlichen Befestigung des Objekts im Abscheider 3, nachdem es in den Abscheider 3 geraten ist, durch die Aufschiebung des Drahtführers 2 auf den Abscheider 3 mit dem Objekt.

Eine Besonderheit der nicht beanspruchten Ausführungsvariante der Einrichtung, die in Fig. 4 dargestellt ist, ist die Möglichkeit der unabhängigen Änderung der Größe des Abscheiders und des Platzes der Platzierung der Netzoberfläche auf dem Abscheider durch die Änderung der Längsplatzierung von zwei Drähten des Drahtführers 2 zueinander.

Eine Besonderheit der Ausführungsvariante der Einrichtung, die in Fig. 5 dargestellt ist, ist die Darstellung der Möglichkeit der Fertigung des Abscheiders 3 und des Katheters 1 (Fig. 5a) und entsprechend des Abscheiders 3 und des geschlossenen Drahtführers 2 (Fig. 5b) aus einem Katheter und den Drähten.

Eine Besonderheit der Ausführungsvariante der Einrichtung, die in Fig. 6 dargestellt ist, ist die einfache Fertigung aus einem Detail (Rohr) und die "absolute" Sicherheit der Verbindung aller Teile der Einrichtung, und zwar des Abscheiders 3, des Katheters 1 und des Drahtführers 2, die zu einem Ganzen gehören.

Eine Besonderheit der Ausführungsvariante der Einrichtung, die in Fig. 7 dargestellt ist, ist die Fertigung der Einrichtung aus einem einfachen Detail, einem einadrigen Draht, und die "absolute" Sicherheit der Verbindung aller Teile der Einrichtung, des Abscheiders 3, des Katheters 1 und des Drahtführers 2, die zu einem Ganzen gehören.

Eine Besonderheit der Ausführungsvariante der Einrichtung, die in Fig. 8 dargestellt ist, ist die Möglichkeit der Bildung eines Abscheiders 3 mit einer Netzoberfläche, die in Fig. 8b dargestellt ist, aus einem mehradrigen mittleren Teil 8 (Fig. 8a).

Eine Besonderheit der Einrichtung, die in Fig. 9 dargestellt ist, ist die Darstellung der Bildung des geschlossenen und des nicht ausgehöhlten Drahtführers 2.

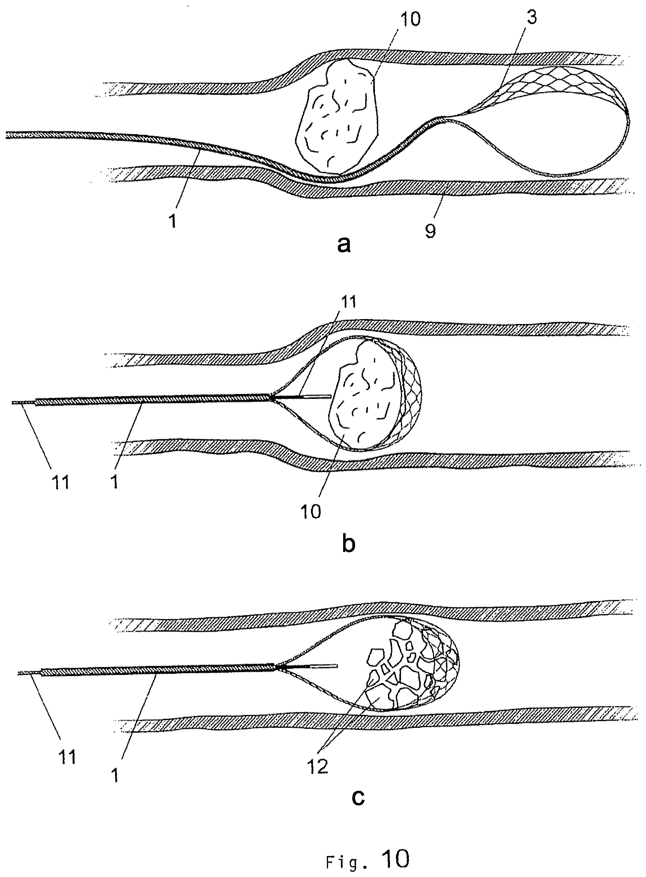

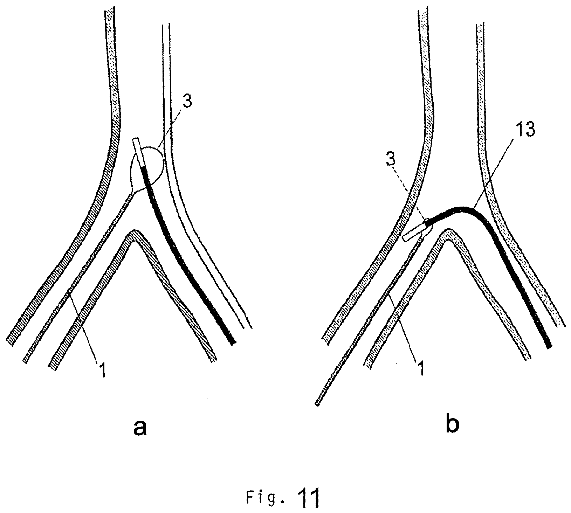

Die Einrichtung funktioniert in folgender Weise:
Durch den Instrumentalkanal des Endoskops wird die Einrichtung mit dem geschlossenen Abscheider des Objekts 3 in den Harnleiter 9 (Fig. 10a) eingeführt, danach werden folgende Manipulationen mit dem Fremdkörper, z. B. mit dem Harnstein 10 durchgeführt. Bei Bedarf wird Kontrastmittel durch den Lumen des ausgehöhlten Drahtführers für die Positionierung des Steins eingeführt. Beim Ausstoßen des Drahtführers aus dem Katheter 1 wird der Abscheider 3 des Objekts in die offene Arbeitsposition gebracht (Fig. 10a). Danach wird der Abscheider 3 in die Position des Greifens des Steins in die Schlinge des Abscheiders gebracht. Mit der Einziehung des Drahtführers 2 in den Katheter 1 wird die Netzoberfläche des Abscheiders nach hinten zum Stein (Fig.10c) gebracht. Der in den Abscheider gebrachte Stein wird zusammen mit der Einrichtung herausgezogen. Wenn es wegen der Größe des Steins oder seiner komplizierten Form nicht klappt, ihn durch eine einmalige Traktion herauszuziehen, wird die Sonde des Lithotripters 11 durch den Lumen des ausgehöhlten Drahtführers bis zum Anschlag in den Stein 10 (Fig. 10b) eingeführt und die Kontakt-Lithotripsie im Körbchen bis zur Bildung von Steinfragmenten (Fig. 10c) durchgeführt. Die im Abscheider 3 gesammelten Steinfragmente 12 werden entfernt. Das Vermahlen von Objekten, die nicht so hart wie Harnsteine sind, kann als mechanische Fragmentierung mit den Seiten des Abscheiders (Gallensteine) oder mit der Unterdruckabsaugung (Thromben, Blutgerinnselbildungen) durchgeführt werden. Die angebotene Einrichtung kann für die Entfernung nicht nur natürlicher Fremdkörper wie Steine, Thromben, Polypen, Tumore, usw. sondern auch für Objekte des "menschlichen Versagens" wie Münzen, Kügelchen, Nüssen, Fetzen des Katheters und der Drähte usw. genutzt werden. Die angebotene Einrichtung kann für die Einstellung und bei Bedarf für die nachfolgende Entfernung der Fremdkörper aus dem Kunststoff, wie z. B., Stents in den Hohlorganen (in den Beispielen nicht gezeigt) sowie für die Versetzung des Fremdkörpers, z. B., der Sonde 13 aus einem Teil des Hohlorgans in den anderen angewendet werden (Fig. 11a, b). Die nicht beanspruchte

Anwendung der lösbaren und unlösbaren Verbindungen des Abscheiders mit Katheter und/oder Drahtführer, der ausgehöhlten und geschlossenen, ein- und mehradrigen Drahtführer und verschiedener Typen der Abscheider ermöglicht dem Betreiber, eine optimale Variante der Ausführung der Einrichtung abhängig von der bestimmten OP-Situation auszuwählen.

Die Industrieformgestaltung, Materialien und Technik der Fertigung der Teile und der Einrichtung im Ganzen beschränken sich nicht auf die beschriebenen Beispiele. Bei der praktischen Umsetzung der angebotenen Einrichtung im Rahmen der angemeldeten Kennzeichnungsmerkmale und bei der Herstellung der bestimmten Varianten der Ausführung der Einrichtung können sämtliche den Fachleuten bekannte Materialien und Verfahren der Fertigung des Katheters, des Abscheiders des Objekts, des Drahtführers und ihrer gegenseitigen Verbindungen angewendet werden. Die vorliegende Einrichtung kann für das Herausziehen von Fremdkörpern aus Hohl- und Rohrorganen, z. B. Harn-, Gallensteinen, Thromben und Polypen, sowie auch für weitere Manipulationen mit Fremdkörpern umfassend angewendet werden.

Der Schutzumfang wird ausschließlich durch die folgenden Ansprüche definiert.

## Patentansprüche

1. Einrichtung für Manipulationen mit Fremdkörpern in Hohlorganen, die einen Katheter (1), Drahtführer (2) und Abscheider (3) eines Objekts enthält, wobei der Abscheider (3) des Objekts mit einer Netzoberfläche mindestens in einem Teil des Abscheiders (3) ausgeführt ist und wobei der Drahtführer (2) ausgehöhlt mit einem Lumen in seinem Inneren als Kanal jeder Form oder geschlossen und nicht ausgehöhlt ohne Lumen ausgeführt ist,
**dadurch gekennzeichnet,**
**dass** der Abscheider (3) des Objekts, der Katheter (1) und der Drahtführer (2) aus einem einzigen Ausgangsdetail aus Metall, Kunststoff oder einem Verbundmaterial in Form eines Rohrs, Drahts oder Bands ausgeführt ist.

2. Einrichtung nach Anspruch 1,
**dadurch gekennzeichnet,**
**dass** das Ausgangsdetail aus Metall, wie zum Beispiel Nitinol, Nirostahl, röntgenkontrastierender Legierung, ausgeführt ist.

3. Einrichtung nach Anspruch 1,
**dadurch gekennzeichnet,**
**dass** das Ausgangsdetail aus Kunststoff, wie zum Beispiel Polyamid, Kapron, Nylon, ausgeführt ist.

## Claims

1. A device for manipulations with foreign bodies in hollow organs, comprising a catheter (1), wire guide (2) and separator (3) of an object, wherein the separator (3) of the object is made with a net surface at least in a part of the separator (3), and wherein the wire guide (2) is made hollow with a lumen in its interior as a channel of any shape or closed and not hollow without lumen,
**characterised in that**
**in that** the separator (3) of the object, the catheter (1) and the wire guide (2) are made from a single initial detail of metal, plastic or a composite material in the form of a tube, wire or strip.

2. Device according to claim 1,
**characterised in that**
that the initial detail is made of metal, such as nitilon, stainless steel, X-ray contrasting alloy.

3. Device according to claim 1,
**characterised in that**
that the initial detail is made of plastic, such as polyamide, kapron, nylon.

## Revendications

1. Dispositif pour les manipulations de corps étrangers dans des organes creux contenant un cathéter (1), un guide-fil (2) et un séparateur (3) d'un objet où le séparateur (3) de l'objet est exécutable à l'aide d'une surface en réseau au minimum dans une partie du séparateur (3) et où le guide-fil (2) vidé avec un lumen dans son intérieur en tant que canal toute forme ou fermé et non vide sans lumen est caractérisé de sorte que le séparateur (3) de l'objet est exécutable par le biais du cathéter (1) et/ou par le biais d'un guide-fil (2) d'un seul détail de départ de métal, de matière synthétique ou d'un matériau composite sous forme de tuyau, de fil ou de bande.

2. Dispositif selon la revendication 1 est caractérisé de sorte que le détail de départ du métal est exécutable à titre d'exemple de nitinol, d'acier niro et d'alliage étant en contraste radiographique.

3. Dispositif selon la revendication 1 est caractérisé de sorte que le détail de départ est exécutable par le biais d'une matière synthétique à titre d'exemple de polyamide, de kaprun et de nylon.
